# EUROPEAN PATENT APPLICATION

(11) **EP 4 467 570 A1**
(43) Date of publication of application: **27.11.2024**
(21) Application number: 22914333.4
(22) Date of filing: 16.12.2022
(51) Int. Cl.: C07K 16/46, C12N 15/62, C12N 15/63, A61K 39/395, A61P 37/08

(54) **FUSION PROTEIN, AND PREPARATION METHOD THEREFOR AND USE THEREOF**

(30) Priority: 31.12.2021 CN 202111673917
(71) Applicant: Zhu, Daocheng, Pudong, Shanghai 201203 (CN)
(72) Inventor: Zhu, Daocheng, Pudong, Shanghai 201203 (CN)
(74) Representative: Michalski Hüttermann & Partner Patentanwälte mbB
(86) International application number: PCT/CN2022/139667
(87) International publication number: WO 2023/125079

(57) **Abstract**

Disclosed are a fusion protein, and a preparation method therefor and a use thereof. In addition, also disclosed are a gene encoding the fusion protein, a method for preparing the fusion protein, an anti-allergy drug using the fusion protein as an active component, and a use of the fusion protein. The fusion protein provided in the present invention is a protein having an amino acid residue as shown in SEQ ID NQ: 3. The fusion protein of the present invention effectively inhibits cellular allergic reaction by enabling an inhibitory system of the allergic reaction in an intracellular signaling system, and will play an important role in the treatment of allergic diseases.

## Description

The present application claims the right of priority for Chinese Patent Application No. 2021116739174 with the filling date of 31 December 2021. This Chinese patent application is incorporated herein by reference in its entirety.

### TECHNICAL FIELD

The present disclosure belongs to the fields of genetic engineering and immunology. In particular, the present disclosure relates to a fusion protein, a gene encoding the fusion protein, a method for preparing the fusion protein, an anti-allergy drug using the fusion protein as an active component, and the use of the fusion protein.

### BACKGROUND

Allergic diseases are the sixth leading cause of acute and chronic diseases, with a clear genetic predisposition. Common allergic diseases are allergic asthma, allergic rhinitis, pollinosis, allergic dermatitis, allergic arthritis, urticaria, allergic shock, etc. About 10%-15% of Chinese and about 20% of Americans are afflicted with allergic diseases.

Currently, drugs used to treat allergic diseases are mostly to control clinical symptoms, such as steroid hormones, antihistamines, decongestants, sodium tryptophan and bronchodilators. Recently, the US FDA approved clinical trials of anti-IgE monoclonal antibodies. However, all of these drugs have varying degrees of side effects, so many drug research and development agencies and pharmaceutical companies at home and abroad are working to find a new anti-allergy drug.

The mechanism of allergic reactions is as follows: antigens (allergens) enter the body and bind to IgE molecules (cytophilic antibodies) attached to mast cells and basophils, which triggers the activation of the immunoreceptor tyrosine-based activation motifs (ITAMs) of the cells to prompt the release of bioactive substances from the cells, causing clinically pathological changes such as smooth muscle contraction, increased vascular permeability and increased serum secretion. In this pathological process, the affinity IgE receptor (FcεRI) on the mast cells and basophils in the body plays a key role in allergic diseases. At the same time, when two molecules of IgG bind to the inhibitory receptor FcyRIIB expressed on the cell membrane surfaces of mast cells and basophils, aggregation occurs, so as to initiate the phosphorylation of the immunoreceptor tyrosine-based inhibition motifs (ITIMs) and inhibit the activation signaling of FcεRI. Studies have been shown that cross-linking of the receptor FcγRII with the receptor FcεRI can induce inhibitory signals in mast cells or basophils, blocking intracellular activation pathways, thereby inhibiting the release of active mediators, further inhibiting allergic reactions (Zhao W, Kepley CL, Morel PA, et al., FcyRIIa, Not FcyRIIb, Is Constitutively and Functionally Expressed on Skin-Derived Human Mast Cells. The Journal of Immunology, 2006, 177(1): 694-701). Therefore, using this mechanism of reaction to study a fusion protein having the ability to activate inhibitory signals in allergic reactions should be a new approach to the treatment of allergies.

Recent studies show that due to the polymorphism of human FcyRII gene expression, a small number of human subcutaneous mast cells do not express FcyRIIB but express FcyRIIA. The intracellular end of the receptor FcyRIIA contains an immunoreceptor tyrosine-based activation motif (ITAM). Direct cross-linking of FcγRIIA with FcεRI (resulting in fusion protein FP4) can cause a phosphorylation in cells involved in allergic reactions so as to activate the activation pathways in the cells, thereby leading to the allergic reaction of cellular degranulation, resulting in allergic diseases. However, the fusion protein FP4 fails to overcome the allergic reactions caused by FcyRIIA expression.

### CONTENT OF THE PRESENT INVENTION

To overcome the defect in the prior art that the binding of the fusion protein FP4 to the receptor FcγRIIA causes the allergic reaction of cellular degranulation, the present disclosure provides a fusion protein, and a preparation method therefor and the use thereof, wherein the fusion protein binds only to the receptor FcyRIIB, with an obviously better affinity than that of the fusion protein FP4 (amino acid sequence as shown in SEQ ID NQ: 1; the DNA sequence encoding same as shown in SEQ ID NQ: 2) and with an unchanged affinity to the IgE receptor FcεRI.

The fusion protein provided by the present disclosure, known as MEG, is a protein having an amino acid residue as shown in SEQ ID NQ: 3, or a protein derived from SEQ ID NO: 3 which is obtained by performing substitution or deletion of one or more amino acid residues on the amino acid residue sequence of SEQ ID NO: 3 and has an activity the same as or better than that of the amino acid residue sequence of SEQ ID NO: 3.

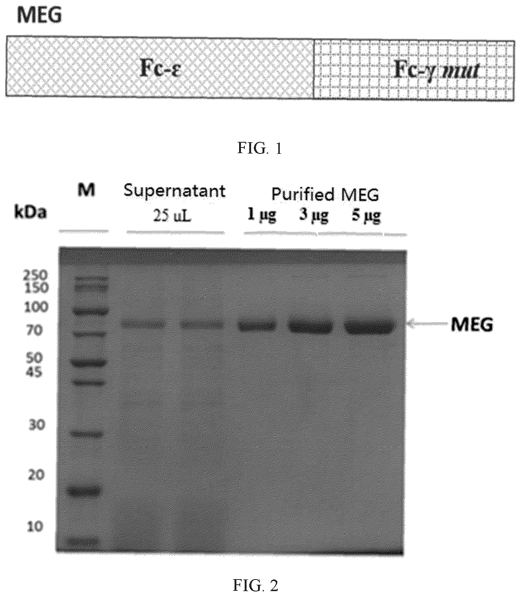

The amino acid residue sequence of SEQ ID NO: 3 is a protein consisting of 552 amino acid residues. The structure of the MEG is as shown in FIG. 1 and consists of two parts: region A (Fc-ε) (amino acid residues 1-320 from the amino terminus) and region B (Fc-γ) (amino acid residues 321-552 from the amino terminus), wherein the region A is derived from a human immunoglobulin IgE, having a site for binding to the IgE receptor FcεRI; the region B is derived from a human immunoglobulin IgG, having a site for binding to the IgG receptor FcyRII.

The technical solutions of the present disclosure are described as follows:
The first technical solution provided by the present disclosure is: a fusion protein, having an amino acid sequence as shown in SEQ ID NO: 3.

The second technical solution provided by the present disclosure is: a gene encoding a fusion protein, the nucleotide sequence of the gene is selected from:
(1) SEQ ID NO: 4;
(2) a polynucleotide encoding the fusion protein of the first technical solution.

In some preferred embodiments, the nucleotide sequence of the gene is as shown in SEQ ID NO: 4.

The third technical solution provided by the present disclosure is: a recombinant expression vector comprising the gene of the second technical solution.

In some preferred embodiments, the backbone plasmid for the recombinant expression vector is pSecTag.

The fourth technical solution provided by the present disclosure is: a transformant comprising the recombinant expression vector of the third technical solution; preferably, the host of the transformant is a mammalian cell.

As a preferred embodiment, the mammalian cell is a CHO cell.

The fifth technical solution provided by the present disclosure is: a method for preparing the fusion protein of the first technical solution, wherein the method comprises culturing the transformant of the fourth technical solution to enable the transformant to express the fusion protein; preferably, the protein is isolated and purified by affinity chromatography; more preferably, the affinity chromatography is an affinity chromatography using murine anti-human IgE.

The sixth technical solution provided by the present disclosure is: a drug, wherein the active component of the drug comprises the fusion protein of the first technical solution.

The seventh technical solution provided by the present disclosure is: the use of the fusion protein of the first technical solution in the preparation of an anti-allergy drug.

The positive effects of the present disclosure lie in that

99.9% of the components of the fusion protein MEG of the present disclosure is derived from a human immunoglobulin, which therefore enters the human body as a drug without any immunogenicity of a foreign protein. Both *in vivo* and *in vitro* experiments demonstrate that the fusion protein MEG can effectively cross-link FcεRI and FcyRII on the surfaces of mast cells or basophils, thereby inhibiting allergic reactions. The fusion protein MEG of the present disclosure is effective in inhibiting allergic reactions in cells mainly by initiating the inhibitory system of the signaling system in the cells involved in allergic reactions and will play an important role in the treatment of allergic diseases.

### BRIEF DESCRIPTION OF THE DRAWINGS

FIG. 1: Schematic diagram of the structure of fusion protein MEG.
FIG. 2: Comparison of supernatant and purified MEG by SDS-PAGE.
FIG. 3: Identification of the binding of fusion protein MEG to receptor FcεRI.
FIG. 4: Identification of the binding of fusion protein MEG to receptor FcyRIIB.
FIG. 5: Analyses of the binding of fusion protein MEG to receptor FcγRIIA (i.e., FcgRIIA) (A) and receptor FcyRIIB (i.e., FcgRIIB) (B).
FIG. 6: Comprehensive analyses of the affinity of fusion protein MEG for FcyR (i.e., FcgR), with the affinity for FcγRI (i.e., FcgRI), the H subtype (i.e., FcgRIIAH) and R subtype (i.e., FcgRIIAR) of FcyRIIA, FcyRIIB (i.e., FcgRIIB), and the F subtype (i.e., FcgRIIIAF) and V subtype (i.e., FcgRIIIAV) of FcγRIIIA as shown in A-F in FIG. 6, respectively.
FIG. 7: The inhibition of allergen-induced degranulation by fusion protein MEG *in vivo.*

### DETAILED DESCRIPTION OF THE PREFERRED EMBODIMENT

The present disclosure is further described below by way of examples; however, the present disclosure is not limited to the scope of the described examples. For the experimental methods in which no specific conditions are specified in the following examples, selections are made according to conventional methods and conditions or according to the product instructions. Reagents and raw materials used in the present disclosure are all commercially available.

### Example 1 Expression and purification of MEG molecules

The expression of the fusion protein FP4 is as shown in CN1317304C, specifically comprising the following steps:
The following operations were performed according to conventional methods: B lymphocytes were isolated from human peripheral blood, and purified, genomic DNA was extracted, and PCR amplification was performed on genes Fcε and Fcγ with specific primers, wherein the PCR amplification product for Fcε DNA was 1155 bp; and the PCR amplification product for Fcγ DNA was 928 bp. The PCR products were separately cloned into pCR4-TOPO (INVITROGEN, CA) to obtain FPEFc-ε and FPGFc-γ, and nucleotide sequence analysis was performed. After obtaining 100% correct nucleotide sequences, the genes Fcε and Fcγ were digested with restriction enzymes SfiI-BamHI and BamHI-NotI (NEW ENGLAND BIOLABS), respectively. The expression vector pSecTag (INVITROGEN, CA) was digested with restriction enzyme SfiI-NotI, the ligation was then performed to obtain plasmid pSecTagMEG, and nucleotide sequence analysis of the plasmid pSecTagMEG was performed and showed that the sequence of the inserted foreign gene was as shown in SEQ ID NQ: 2. This plasmid was then transfected into mouse myeloma SP2/0 cells by conventional electroporation, and was subjected to ZEOCIN resistance screening and ELISA identification (ELISA plates were coated with a murine anti-human IgE antibody prepared by conventional methods, the supernatant of the sample was added, and then a goat anti-human IgE enzyme-labeled antibody was added) to obtain a clone that highly expresses FP4 (with an amino acid sequence as shown in SEQ ID NQ: 1).

The construction of MEG (see FIG. 1 for the structure of MEG) differs from that of FP4 in that:
(1) MEG gene is synthesized on the basis of human immunoglobulin IgE Fcε and IgG Fcγ sequences, and cloned into a eukaryotic expression vector pBG by SOE PCR and enzyme digestion. After sequencing verification, the correct MEG gene is transfected into CHO cells and expressed in 50% CD CHO+50% Dynamis medium.
(2) A high purity protein is obtained by two-step purification comprising GE Mab-select affinity chromatography and cation exchange, with a purity of greater than 90% as detected by SDS-PAGE (results as shown in FIG. 2) and HPLC. After buffer replacement using ultrafiltration and filtration, the protein samples are quantified.
(3) MEG has an amino acid mutation at the amino acid residue of Fc-γ relative to FP4, from P to D.

Amino acid sequence of FP4: 
FTPPTVKILQSSCDGGGHFPPTIQLLCLVSGYTPGTINITWLEDGQVMDVDLS
TASTTQEGELASTQSELTLSQKHWLSDRTYTCQVTYQGHTFEDSTKKCADSNPRGVS
AYLSRPSPFDLFIRKSPTITCLVVDLAPSKGTVNLTWSRASGKPVNHSTRKEEKQRNG
TLTVTSTLPVGTRDWIEGETYQCRVTHPHLPRALMRSTTKTSGPRAAPEVYAFATPEW
PGSRDKRTLACLIQNFMPEDISVQWLHNEVQLPDARHSTTQPRKTKGSGFFVFSRLE
VTRAEWEQKDEFICRAVHEAASPSQTVQRAVSVNPGKGSEPKSCDKTHTCPPCPAPEL
LGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK (SEQ ID NO: 1)
 Where the underlined line represents region B Fc-γ.
Nucleotide sequence encoding FP4 gene: 
ttcaccccgcccaccgtgaagatcttacagtcgtcctgcgacggcggcgggcacttccccccgaccatccagctcctgtg
cctcgtctctgggtacaccccagggactatcaacatcacctggctggaggacgggcaggtcatggacgtggacttgtccaccgcctct
accacgcaggagggtgagctggcctccacacaaagcgagctcaccctcagccagaagcactggctgtcagaccgcacctacacct
gccaggtcacctatcaaggtcacacctttgaggacagcaccaagaagtgtgcagattccaacccgagaggggtgagcgcctacctaa
gccggcccagcccgttcgacctgttcatccgcaagtcgcccacgatcacctgtctggtggtggacctggcacccagcaaggggacc
 
gtgaacctgacctggtcccgggccagtgggaagcctgtgaaccactccaccagaaaggaggagaagcagcgcaatggcacgttaa
ccgtcacgtccaccctgccggtgggcacccgagactggatcgagggggagacctaccagtgcagggtgacccacccccacctgcc
cagggccctcatgcggtccacgaccaagaccagcggcccgcgtgctgccccggaagtctatgcgtttgcgacgccggagtggccg
gggagccgggacaagcgcaccctcgcctgcctgatccagaacttcatgcctgaggacatctcggtgcagtggctgcacaacgaggt
gcagctcccggacgcccggcacagcacgacgcagccccgcaagaccaagggctccggcttcttcgtcttcagccgtctagaggtga
ccagggccgaatgggagcagaaagatgagttcatctgccgtgcagtccatgaggcagctagcccctcacagaccgtccagcgagc
ggtgtctgtaaatcccggtaaaggatccgagcccaaatcttgtgacaaaactcacacatgcccaccgtgcccagcacctgaactcctg
gggggaccgtcagtcttcctcttccccccaaaacccaaggacaccctcatgatctcccggacccctgaggtcacatgcgtggtggtgg
acgtgagccacgaagaccctgaggtcaagttcaactggtacgtggacggcgtggaggtgcataatgccaagacaaagccgcggga
ggagcagtacaacagcacgtaccgggtggtcagcgtcctcaccgtcctgcaccaggactggctgaatggcaaggagtacaagtgca
aggtctccaacaaagccctcccagcccccatcgagaaaaccatctccaaagccaaagggcagccccgagaaccacaggtgtacac
cctgcccccatcccgggatgagctgaccaagaaccaggtcagcctgacctgcctggtcaaaggcttctatcccagcgacatcgccgt
ggagtgggagagcaatgggcagccggagaacaactacaagaccacgcctcccgtgctggactccgacggctccttcttcctctaca
gcaagctcaccgtggacaagagcaggtggcagcaggggaacgtcttctcatgctccgtgatgcatgaggctctgcacaaccactaca
cgcagaagagcctctccctgtctccgggtaaatga
**[0045]** Amino acid sequence of fusion protein MEG: 
FTPPTVKILQSSCDGGGHFPPTIQLLCLVSGYTPGTINITWLEDGQVMDVDLS
TASTTQEGELASTQSELTLSQKHWLSDRTYTCQVTYQGHTFEDSTKKCADSNPRGVS
AYLSRPSPFDLFIRKSPTITCLVVDLAPSKGTVNLTWSRASGKPVNHSTRKEEKQRNG
TLTVTSTLPVGTRDWIEGETYQCRVTHPHLPRALMRSTTKTSGPRAAPEVYAFATPEW
PGSRDKRTLACLIQNFMPEDISVQWLHNEVQLPDARHSTTQPRKTKGSGFFVFSRLE
VTRAEWEQKDEFICRAVHEAASPSQTVQRAVSVNPGKEPKSCDKTHTCPPCPAPELL
GGDSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK (SEQ ID NO: 3)
 Where the underlined line represents region B Fc-γ.
Nucleotide sequence of *MEG*:

ttcaccccgcccaccgtgaagatcttacagtcgtcctgcgacggcggcgggcacttccccccgaccatccagctcctgtg
cctcgtctctgggtacaccccagggactatcaacatcacctggctggaggacgggcaggtcatggacgtggacttgtccaccgcctct

accacgcaggagggtgagctggcctccacacaaagcgagctcaccctcagccagaagcactggctgtcagaccgcacctacacct
gccaggtcacctatcaaggtcacacctttgaggacagcaccaagaagtgtgcagattccaacccgagaggggtgagcgcctacctaa
gccggcccagcccgttcgacctgttcatccgcaagtcgcccacgatcacctgtctggtggtggacctggcacccagcaaggggacc
gtgaacctgacctggtcccgggccagtgggaagcctgtgaaccactccaccagaaaggaggagaagcagcgcaatggcacgttaa
ccgtcacgtccaccctgccggtgggcacccgagactggatcgagggggagacctaccagtgcagggtgacccacccccacctgcc
cagggccctcatgcggtccacgaccaagaccagcggcccgcgtgctgccccggaagtctatgcgtttgcgacgccggagtggccg
gggagccgggacaagcgcaccctcgcctgcctgatccagaacttcatgcctgaggacatctcggtgcagtggctgcacaacgaggt
gcagctcccggacgcccggcacagcacgacgcagccccgcaagaccaagggctccggcttcttcgtcttcagccgcctggaggtg
accagggccgaatgggagcagaaagatgagttcatctgccgtgcagtccatgaggcagctagcccctcacagaccgtccagcgag
cggtgtctgtaaatcccggtaaagagcccaaatcttgtgacaaaactcacacatgcccaccgtgcccagcacctgaactcctgggggg
agactcagtcttcctcttccccccaaaacccaaggacaccctcatgatctcccggacccctgaggtcacatgcgtggtggtggacgtg
agccacgaagaccctgaggtcaagttcaactggtacgtggacggcgtggaggtgcataatgccaagacaaagccgcgggaggagc
agtacaacagcacgtaccgtgtggtcagcgtcctcaccgtcctgcaccaggactggctgaatggcaaggagtacaagtgcaaggtct
ccaacaaagccctcccagcccccatcgagaaaaccatctccaaagccaaagggcagccccgagaaccacaggtgtacaccctgcc
cccatcccgggatgagctgaccaagaaccaggtcagcctgacctgcctggtcaaaggcttctatcccagcgacatcgccgtggagtg
ggagagcaatgggcagccggagaacaactacaagaccacgcctcccgtgctggactccgacggctccttcttcctctacagcaagct
caccgtggacaagagcaggtggcagcaggggaacgtcttctcatgctccgtgatgcatgaggctctgcacaaccactacacgcaga
agagcctctccctgtctccgggtaaatga (SEQ ID NO: 4)

### Example 2 Assay of the binding of MEG to receptors FcεRI and FcyRII, respectively

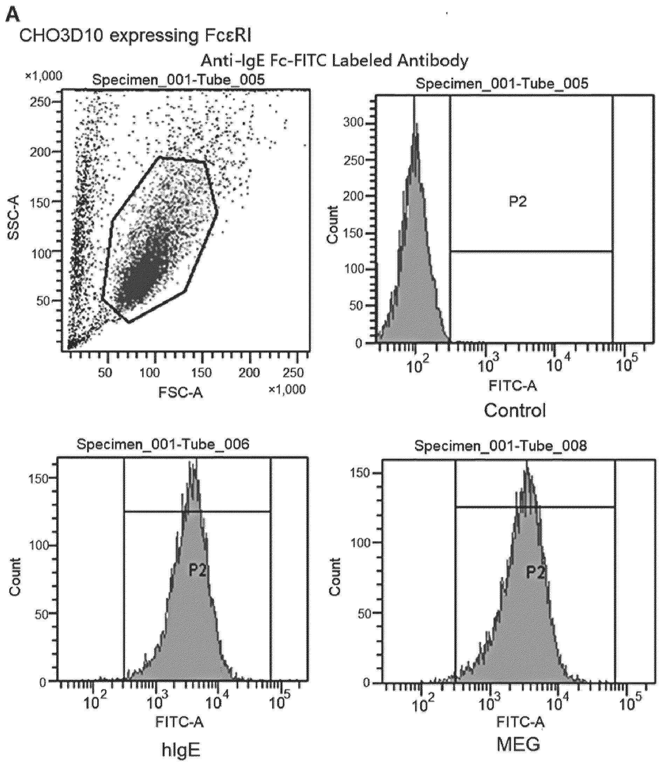

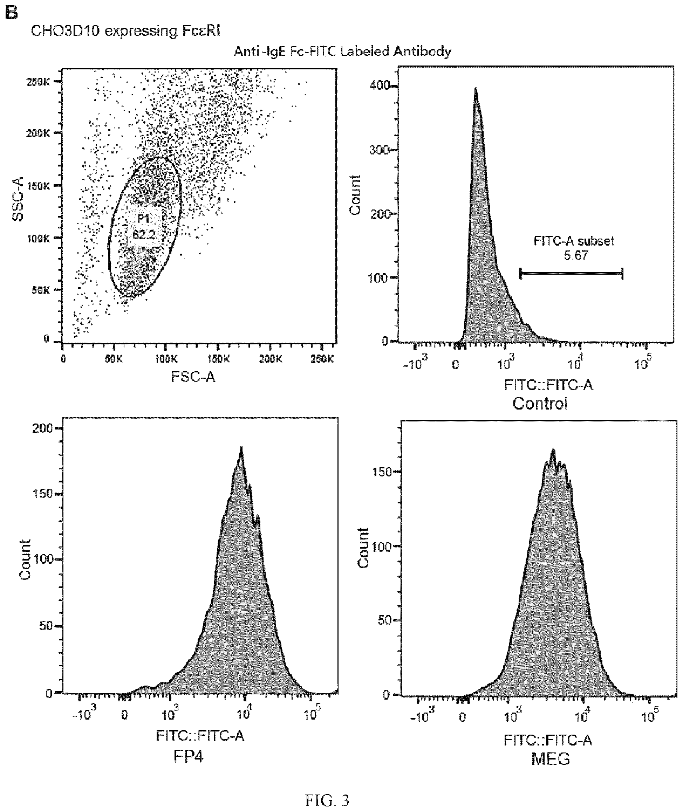

Assay of binding to receptor FcεRI: 1 × 10⁶ CHO 3D10 cells (expressing receptor FcεRI) were separately reacted with 5 µg of hIgE, 5 µg of FP4 and 5 µg of MEG protein purified in Example 2 at 4 °C for 1 hour. After washing, 5 µL of anti-human IgE Fc-FITC labeled antibody (CALTAG, CA) was added, and the cells were detected by a flow cytometer. The results show that the MEG protein can bind to the receptor FcεRI compared with hIgE (A in FIG. 3) and FP4 (B in FIG. 3).

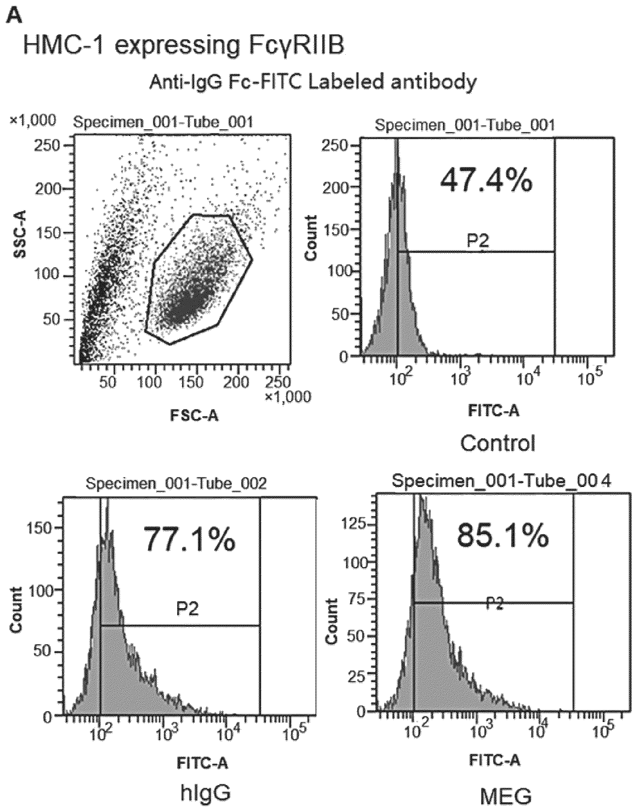

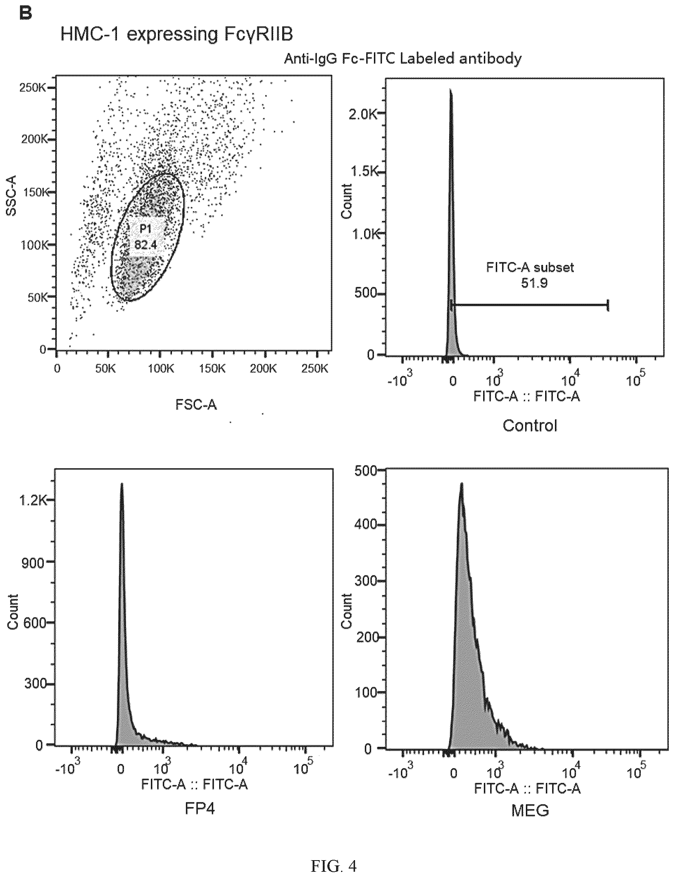

Assay of binding to receptor FcyRII: 1 × 10⁶ HMC-1 cells (expressing receptor FcγRIIB) were separately reacted with 5 µg of hIgG, 5 µg of FP4 and 5 µg of MEG protein purified in Example 2 at 4 °C for 1 hour. After washing, 5 µL of anti-human IgG Fc-FITC labeled antibody (CALTAG, CA) was added, and the cells were detected by a flow cytometer. The results show that the MEG protein can bind to the receptor FcyRIIB compared with hIgG (A in FIG. 4) and FP4 (B in FIG. 4).

### Example 3 ELISA analysis of properties of MEG of binding to different types of FcyR receptors

100 µL of MEG at an appropriate concentration (2 µg/mL) was added to each well of enzyme-linked plates for coating overnight, the supernatant was discarded, blocking was performed with PBS containing 1% BSA for 2 hours, the plates were washed twice with PBST (PBS containing 0.05% Tween 20), extracellular domain proteins such as FcyRI, FcγRIIAH, FcgRIIAR, FcgRIIIAF, FcgRIIIAV or FcyRIIB tagged with biotin (Sino Biological Inc.) at an appropriate concentration were added separately, incubation was performed at room temperature for 1 hour, and then the supernatant was discarded. The plates were washed three times with PBST. Streptavidin-HRP (BD Biosciences) was added, incubation was performed at room temperature for 1 hour, and biotin protein was detected. The supernatant was removed, and the plates were washed four times with PBST. A color development solution was added for color development for 5-40 minutes, and then the absorbance A650 at 650 nm was measured.

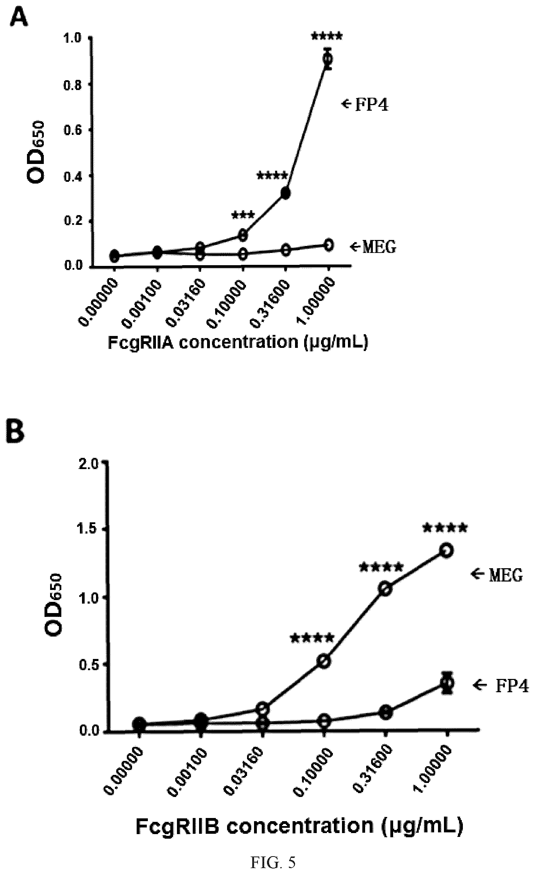

In the first round, the results show that the MEG protein does not bind to FcγRIIAH (i.e., FcgRIIA as shown in A in FIG. 5), but only to receptor FcyRIIB (i.e., FcgRIIB), and has an obviously better affinity for FcyRIIB than FP4 (as shown in B in FIG. 5).

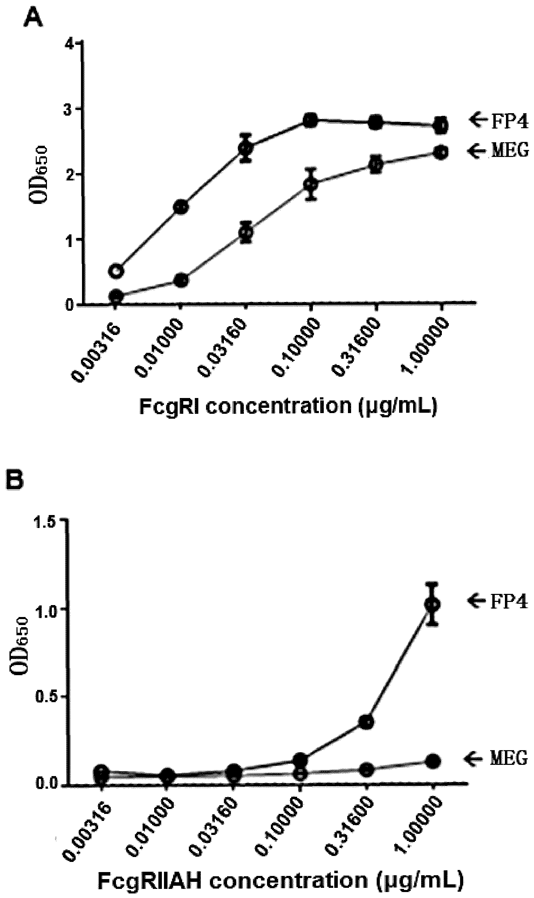

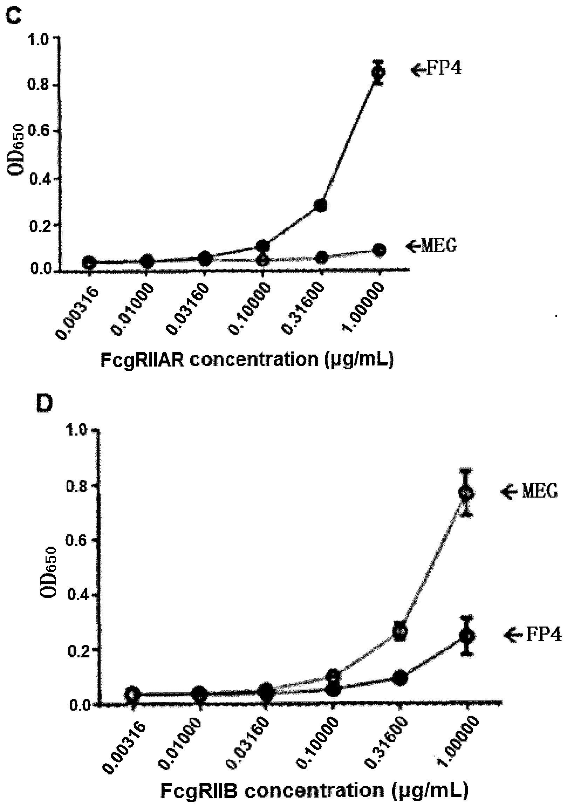

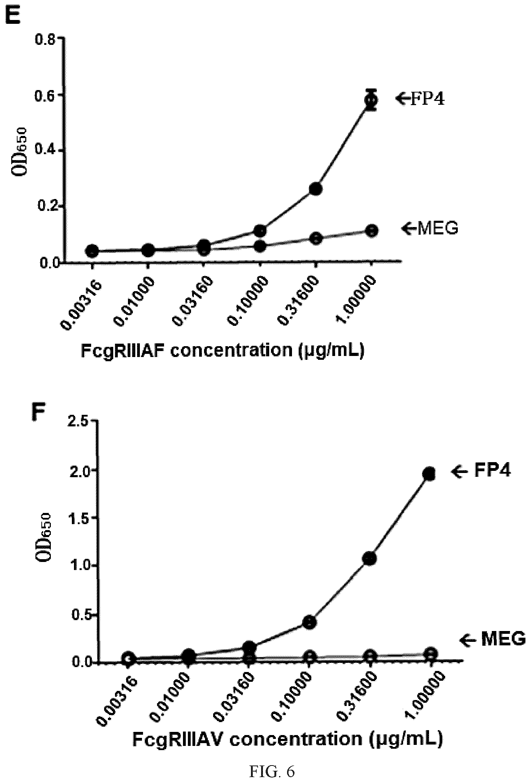

In the second round, different subtypes of FcyRI, FcγRII and FcyRIII were further analyzed comprehensively. The results show that the MEG protein does not bind to FcγRI (i.e., FcgRI) (A in FIG. 6), H subtype (i.e., FcgRIIAH) (B in FIG. 6) and R subtype (i.e., FcgRIIAR) (C in FIG. 6) of FcyRIIA, as well as F subtype (i.e., FcgRIIIAF) (E in FIG. 6) and V subtype (i.e., FcgRIIIAV) (F in FIG. 6) of FcγRIIIA, only to receptor FcyRIIB (i.e., FcgRIIB), and has an obviously better affinity for FcyRIIB than FP4 (D in FIG. 6).

### Example 4 Inhibition of allergen-induced degranulation by MEG protein in vivo

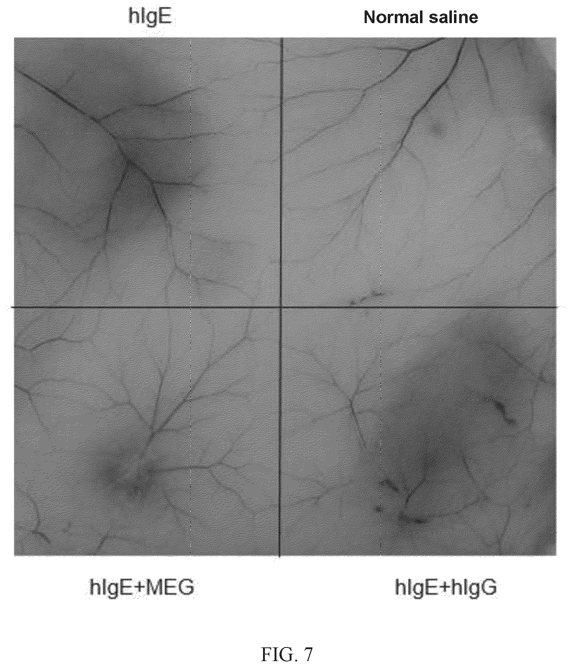

A specific human anti-NP-IgE (Serotech) was injected subcutaneously into transgenic mice (with knockout of murine IgE receptor FcεRI α-chain and insertion of human IgE receptor FcεRI α-chain). 4 hours later, 100 µg of allergen NP and 1% Evans Blue (EVANS) dye were injected i.v.. When an allergic reaction of degranulation occurs at the sensitized site, due to the increased vascular permeability of the local skin, the blue dye leaks from the blood vessels into the interstitial spaces, and the subcutaneous mucosa shows a blue reaction. When an equivalent amount of MEG protein is added at the same time as the corresponding skin site is sensitized, the allergic reaction of degranulation is completely inhibited, without any reaction in the local skin, while the addition of the equivalent amount of human IgG control shows no inhibition (as shown in FIG. 7).

## Claims

1. A fusion protein, wherein the amino acid sequence of the fusion protein is as shown in SEQ ID NO: 3.

2. A gene encoding a fusion protein, wherein the nucleotide sequence of the gene is selected from:
1) SEQ ID NO: 4;
2) a polynucleotide encoding the fusion protein according to claim 1.

3. The gene according to claim 2, wherein the nucleotide sequence of the gene is as shown in SEQ ID NO: 4.

4. A recombinant expression vector, wherein the recombinant expression vector comprises the gene according to claim 2 or 3.

5. The recombinant expression vector according to claim 4, wherein the backbone plasmid of the recombinant expression vector is pSecTag.

6. A transformant, wherein the transformant comprises the recombinant expression vector according to claim 4 or 5; preferably, the host of the transformant is a mammalian cell.

7. The transformant according to claim 6, wherein the mammalian cell is a CHO cell.

8. A method for preparing the fusion protein according to claim 1, wherein the method comprises culturing the transformant according to claim 6 or 7 to enable the transformant to express the fusion protein; preferably, the fusion protein is isolated and purified by affinity chromatography; more preferably, the affinity chromatography is an affinity chromatography using murine anti-human IgE.

9. A drug, wherein the active component of the drug comprises the fusion protein according to claim 1.

10. Use of the fusion protein according to claim 1 in the preparation of an anti-allergy drug.
